(19) Europäisches Patentamt
European Patent Office
Office européen des brevets

(11) **EP 1 893 558 B1**

(12) **EUROPÄISCHE PATENTSCHRIFT**

(45) Veröffentlichungstag und Bekanntmachung des Hinweises auf die Patenterteilung:
**06.06.2018 Patentblatt 2018/23**

(51) Int Cl.:
***C07C 67/08*** *(2006.01)*     ***C07C 69/75*** *(2006.01)*
***C08K 5/12*** *(2006.01)*     ***C07C 67/303*** *(2006.01)*

(21) Anmeldenummer: **06754898.2**

(22) Anmeldetag: **27.04.2006**

(86) Internationale Anmeldenummer:
**PCT/EP2006/061889**

(87) Internationale Veröffentlichungsnummer:
**WO 2006/136471 (28.12.2006 Gazette 2006/52)**

(54) **GEMISCH VON DIISONONYLESTERN DER 1,2-CYCLOHEXANDICARBONSÄURE, VERFAHREN ZU DEREN HERSTELLUNG UND VERWENDUNG DIESER GEMISCHE**

MIXTURE OF DIISONONYL ESTERS OF 1,2-CYCLOHEXANEDICARBOXYLIC ACID, METHOD FOR THE PRODUCTION THEREOF AND USE OF THESE MIXTURES

MELANGE D'ESTERS DIISONONYLIQUES DE L'ACIDE 1,2- CYCLOHEXANDICARBOXYLIQUE, PROCEDE POUR PRODUIRE CES MELANGES ET UTILISATION ASSOCIEE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.06.2005 DE 102005028752**

(43) Veröffentlichungstag der Anmeldung:
**05.03.2008 Patentblatt 2008/10**

(73) Patentinhaber: **Evonik Degussa GmbH**
**45128 Essen (DE)**

(72) Erfinder:
• **GRASS, Michael**
**45721 Haltern am See (DE)**
• **LANG, Arne**
**45770 Marl (DE)**

(56) Entgegenhaltungen:
**EP-A- 1 314 772     EP-A- 1 475 071**
**WO-A-2004/009526     WO-A-2004/081127**
**WO-A1-03/029180     DE-A1- 10 321 100**
**US-A1- 2003 069 135**

**Beschreibung**

Die vorliegende Erfindung betrifft ein Verfahren zur Herstellung von Gemischen von Diisononylestern der 1,2-Cyclohexandicarbonsäure.

Alicyclische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Kunststoffe wie Polyvinylchlorid (PVC), Polyvinylbutyral (PVB) und Polyolefine Verwendung.

Für die Weichmachung von PVC werden zur Zeit überwiegend Ester der Phthalsäure, wie beispielweise Dibutyl-, Dioctyl-, Dinonyl- oder Didecylester, verwendet. Da diese Phthalate in letzter Zeit häufig als gesundheitsschädlich bezeichnet werden, muss befürchtet werden, dass deren Einsatz in Kunststoffen eingeschränkt werden könnte. In WO 03/029339 wird beschrieben, dass Di-2-ethylhexylphthalate (DEHP, oft auch als DOP bezeichnet) und Diisononylphthalate (DINP) für die meisten Anwendungen durch Di-2-ethylhexyl-1,2-cyclohexyldicarbonsäureester (DEHCH) und Diisononyl-1,2-cyclohexandicarbonsäureester (DINCH) ersetzt werden können, da die entsprechenden kernhydrierten Ester bezüglich ihrer durch die Shore-Härte ausgedrückten weichmachenden Wirkung ähnliche Eigenschaften aufweisen wie die nicht kernhydrierten Ester mit gleicher Alkoholkomponente. Alicyclische Polycarbonsäureester könnten deshalb als Ersatzstoffe, wenn auch mit einem insgesamt etwas anderen anwendungstechnischen Profil, zum Ersatz der Phthalate zur Verfügung stehen.

Das Dokument WO 2003/029180 A1 offenbart die Herstellung von Phthalaten durch Veresterung von Alkoholen unterschiedlichster Kettenlänge mit Phthalsäure oder Phthalsäureanhydrid.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:
In US 5,286,898 und US 5,319,129 wird ein Verfahren beschrieben, mit dem Dimethylterephthalat an geträgerten Pd-Katalysatoren, die mit Ni, Pt und/oder Ru dotiert sind, bei Temperaturen größer oder gleich 140 °C und einem Druck zwischen 50 und 170 bar zum entsprechenden Hexahydrodimethylterephthalat hydriert werden kann.

In DE 28 23 165 werden aromatische Carbonsäureester an geträgerten Ni, Ru, Rh und/oder Pd-Katalysatoren zu den entsprechenden alicyclischen Carbonsäureestern bei 70 bis 250 °C und 30 bis 200 bar hydriert. US 3,027,398 offenbart die Hydrierung von Dimethylterephthalat an geträgerten Ru-Katalysatoren bei 110 bis 140°C und 3,5 bis 10,5 MPa.

In WO 00/78704 wird ein Verfahren zur Hydrierung von Benzolpolycarbonsäureestern zu den entsprechenden alicyclischen Verbindungen offengelegt. Dabei werden bevorzugt Trägerkatalysatoren eingesetzt, die Ru alleine oder zusammen mit mindestens einem Metall der I., VII. oder VIII. Nebengruppe des Periodensystems enthalten und 5 bis 50 % Makroporen aufweisen.

In DE 101 61 010 ist die Herstellung von Cyclohexan-1,2-dicarbonsäureestern ausgehend von einem Dien/Maleinsäureanhydrid-Gemisch über eine Reaktionssequenz, die eine Diels-Alder-Reaktion umfasst, beschrieben.

In US 2003/0069135 A1 werden Cyclohexan-1,2-dicarbonsäureestern sowohl durch Kernhydrierung als auch durch Butendimerisierung hergestellt.

WO 2004/009526 A1 beschreibt ausführlich feinporige Katalysatoren, welche in der Kernhydrierung aromatischer Polycarbonsäure eingesetzt werden.
Weiterhin können die Cyclohexandicarbonsäureester durch Veresterung von Cyclohexandicarbonsäure oder geeigneten Derivaten mit den entsprechenden Alkoholen erhalten werden.

Häufig werden in der Technik Gemische von isomeren Estern eingesetzt. Ausgehend von EP 1 042 273 oder DE 101 16 812 lag jedoch die Vermutung nahe, dass die diversen dort beanspruchten Cyclohexandicarbonsäurediisononylester, die beispielsweise aus verschiedenen Diisononylphthalaten (DINP) durch Kernhydrierung zugänglich sind, sich in ihren Eigenschaften nicht grundsätzlich unterscheiden. Insbesondere wurden die Hydrierprodukte der DINP-Typen, deren Alkoholketten auf Basis von n-Buten oder iso-Buten erhalten werden können, als geeignet bezeichnet.

Eines der wichtigsten Phthalate ist das Di-2-ethylhexylphthalat (DEHP). Auf Grund einer möglichen Gesundheitsgefährdung, die evtl. von der Verwendung dieses Weichmachers ausgehen kann, wird in sensiblen Anwendungen wie zum Beispiel Medizinalartikel, Spielzeug oder im Lebensmittelkontaktbereich nach Weichmachern gesucht, die die Phthalate, insbesondere das DEHP substituieren könnten.

Die Aufgabe der vorliegenden Erfindung bestand deshalb in der Bereitstellung eines Verfahrens zur Herstellung eines Weichmachers, der anwendungstechnische Eigenschaften aufweist, die es ermöglichen, das DEHP in einigen, vorzugsweise in möglichst vielen Anwendungen, zu ersetzen.

Es wurde gefunden, dass die anwendungstechnischen Eigenschaften der Cyclohexandicarbonsäurediisononylester durch Wahl der Zusammensetzung der Alkoholkomponente so eingestellt werden können, dass sie geeignet sind, DEHP mit möglichst geringem Aufwand zu ersetzen. Es wurde gefunden, dass Gemische von Diisononylestern der 1,2-Cyclohexandicarbonsäure, deren Isononylreste einen Verzweigungsgrad von 1,2 bis 2,0 aufweisen, besonders gut als Ersatz von DEHP als Weichmacher für PVC geeignet sind.

Gegenstand der vorliegenden Erfindung ist ein Verfahren zur Herstellung von Gemischen von Diisononylestern der 1,2-Cyclohexandicarbonsäure gemäß Anspruch 1.

Die hergestellten Gemische haben den Vorteil, dass sie eine niedrige Viskosität in Plastisolen aufweisen. Weiterhin ist der Massenverlust nach Wärmealterung gering.

Außerdem haben die hergestellten Gemische von Cyclohexandicarbonsäurediisononylester (DINCH) den Vorteil, dass sie bezüglich des Kälteflexibilisierungsvermögens ähnliche oder bessere Werte aufweisen, als das DEHP und dass sie bezüglich der meisten Verarbeitungseigenschaften, z. B. in Dry-Blends (Pulvermischungen), ein vom DEHP nur marginal abweichendes Profil aufweisen. Bei einem Ersatz von DEHP durch das hergestellte DINCH können somit die erforderlichen Anpassungen auf ein Minimum begrenzt bleiben.

Nachfolgend wird die Erfindung beispielhaft beschrieben, ohne dass die Erfindung, deren Schutzbereich sich aus den Ansprüchen und der gesamten Beschreibung ergibt, darauf beschränkt sein soll. Auch die Ansprüche selbst gehören zum Offenbarungsgehalt der vorliegenden Erfindung. Sind im nachfolgenden Text Bereiche bzw. Vorzugsbereiche angegeben, so sollen auch alle in diesen Bereichen liegenden, theoretisch möglichen Einzelwerte und Teilbereiche zum Offenbarungsgehalt der vorliegenden Erfindung gehören, ohne dass diese aus Gründen der besseren Übersichtlichkeit explizit genannt worden sind.

Im Rahmen der vorliegenden Erfindung werden, wenn nicht ausdrücklich etwas anderes beschrieben wird, unter 1,2-Cyclohexandicarbonsäure oder deren Ester, cis- oder transisomerenreine Verbindungen oder auch Gemische von cis- und trans-Isomeren dieser Verbindungen verstanden.

Das Gemisch von Diisononylestern der 1,2-Cyclohexandicarbonsäure (DINCH), zeichnet sich dadurch aus, dass die Isononylreste der im Gemisch enthaltenen Diisononylester einen Verzweigungsgrad von 1,2 bis 2,0, vorzugsweise von 1,2 bis 1,9, bevorzugt von 1,3 bis 1,8 und besonders bevorzugt von 1,3 bis 1,7 aufweisen. Die Isononylreste sind dabei solche, die auf primären Isononanolen basieren.

Die Ermittlung des Verzweigungsgrades kann, wenn wie in der vorliegenden Erfindung ausschließlich am Ring unsubstituierte 1,2-Cyclohexandicarbonsäurereste vorliegen, durch $^1$H-NMR- oder $^{13}$C-NMR-Methoden erfolgen. Gemäß der vorliegenden Erfindung erfolgt die Ermittlung des Verzweigungsgrades bevorzugt mit Hilfe der $^1$H-NMR-Spektroskopie an einer Lösung der Diisononylester in Deuterochloroform ($CDCl_3$). Für die Aufnahme der Spektren werden zum Beispiel 20 mg Substanz in 0,6 ml $CDCl_3$ (enthaltend 1 Massen-% TMS) gelöst und in ein NMR-Röhrchen mit einem Durchmesser von 5 mm gefüllt. Sowohl die zu untersuchende Substanz als auch das verwendete $CDCl_3$ können zunächst über Molekularsieb getrocknet werden, um Verfälschungen der Messwerte durch evtl. vorhandenes Wasser auszuschließen. Die Methode der Bestimmung des Verzweigungsgrades ist gegenüber anderen Methoden zur Charakterisierung von Alkoholresten, wie sie z. B. in WO 03/029339 beschrieben werden vorteilhaft, da Verunreinigungen mit Wasser im Wesentlichen keinen Einfluss auf die Messergebnisse und deren Auswertung haben. Prinzipiell können mit der $^1$H-NMR-Spektroskopie die Verzweigungsgrade der primären Isononylreste unabhängig davon bestimmt werden, ob der Säurerest auf einer Phthalsäure oder einer 1,2-Cyclohexancarbonsäure basiert, solange die Säuren keine Substituenten aufweisen, die eine -O-CH2-Gruppe oder eine Methyl-Gruppe enthalten. Die NMR-spektroskopischen Untersuchungen können prinzipiell mit jedem handelsüblichen NMR-Gerät durchgeführt werden. Für die vorliegenden NMR-spektroskopischen Untersuchung wurde ein Gerät vom Typ Avance 500 der Firma Bruker eingesetzt. Die Spektren wurden bei einer Temperatur von 300 K mit einem Delay (Verzögerung) von d1 = 5 Sekunden, 32 Scans (Durchgänge), einer Pulslänge von 9,7 $\mu$s und einer Sweep Width (Spektrale Breite) von 10000 Hz mit einem 5 mm BBO-Probenkopf (broad band observer; Breitbanbeobachtung) aufgenommen. Die Resonanzsignale werden gegen die chemischen Verschiebungen von Tetramethylsilan (TMS = 0 ppm) als internen Standard aufgezeichnet. Mit anderen handelsüblichen NMR-Geräten werden mit den gleichen Betriebsparametern vergleichbare Ergebnisse erhalten.

Die erhaltenen $^1$H-NMR-Spektren der Gemische von Diisononylestern der 1,2-Cyclohexandicarbonsäure weisen im Bereich von 0,5 ppm bis zum Minimum des niedrigsten Tals im Bereich von 0,9 bis 1,1 ppm Resonanzsignale auf, die (im Wesentlichen) durch die Signale der Wasserstoffatome der Methylgruppe(n) der Isononylgruppen gebildet werden. Die Signale im Bereich der chemischen Verschiebungen von 3,6 bis 4,4 ppm können (im Wesentlichen) den Wasserstoffatomen der Methylengruppe, die dem Sauerstoff des Alkohols bzw. des Alkoholrests benachbart ist, zugeordnet werden. Die Quantifizierung erfolgt durch Bestimmung der Fläche unter den jeweiligen Resonanzsignalen, d. h. der vom Signal von der Grundlinie eingeschlossenen Fläche. Handelsübliche NMR-Geräte verfügen über Vorrichtungen zur Integration der Signalfläche. In den vorliegenden NMR-spektroskopischen Untersuchung wurde die Integration mit Hilfe der Software "xwinnmr", Version 3.5 durchgeführt. Anschließend wird der Integralwert der Signale im Bereich von 0,5 bis zum Minimum des niedrigsten Tals im Bereich von 0,9 bis 1,1 ppm durch den Integralwert der Signale im Bereich von 3,6 bis 4,4 ppm dividiert und man erhält so ein Intensitätsverhältnis, dass das Verhältnis der Anzahl der Wasserstoffatome die in einer Methylgruppe vorhanden sind zur Anzahl der Wasserstoffatome, die in einer einem Sauerstoff benachbarten Methylengruppe vorhanden sind, angibt. Da pro Methylgruppe drei Wasserstoffatome vorhanden sind und je einem Sauerstoff benachbarter Methylengruppe zwei Wasserstoffatome vorhanden sind, müssen die Intensitäten jeweils durch 3 bzw. 2 geteilt werden um das Verhältnis der Anzahl der Methylgruppen im Isononylrest zur Anzahl der einem Sauerstoff benachbarten Methylengruppe im Isononylrest zu erhalten. Da ein lineares primäres Nonanol, welches nur eine Methylgruppe und eine einem Sauerstoff benachbarter Methylengruppe aufweist, keine Verzweigung enthält und demnach einen Verzweigungsgrad von 0 aufweisen muss, muss von dem Verhältnis noch der Betrag 1 subtrahiert werden.

Der Verzweigungsgrad V kann also gemäß folgender Formel I aus dem gemessenen Intensitätsverhältnis berechnet werden:

$$V = 2/3 * I(CH_3)/I(OCH_2) - 1 \qquad\qquad \textbf{I}$$

Mit V = Verzweigungsgrad, $I(CH_3)$ = Flächenintegral, welches im Wesentlichen den Methylwasserstoffatomen zugeordnet ist, und $I(OCH_2)$ = Flächenintegral der Methylenwasserstoffatome in Nachbarschaft zum Sauerstoff.

Vorzugsweise weisen Nonylalkohole, die durch Verseifung der Diisononylester erhalten werden, weniger als 10 Mol-%, bevorzugt weniger 5 Mol-%, besonders bevorzugt weniger als 1 Mol-% und ganz besonders bevorzugt von 0,5 bis 0,0001 Mol-% an 3,5,5-Trimethylhexanol auf. Die Verseifung der Diisononylester kann nach üblichen Methoden durch Umsetzung mit alkalischen Medien erfolgen (siehe z. B. Ullmann's Enzyklopädie der Technischen Chemie, 5 Ed. A 10, S. 254-260, 1986). Die Bestimmung des Anteils an 3,5,5-Trimethylhexanol kann auf übliche Weise durch gaschromatographische Analysemethoden (GC) erfolgen.

Das Gemisch von Diisononylestern der 1,2-Cyclohexandicarbonsäure wird durch Hydrierung von Diisononylphthalaten erhalten. Die Hydrierung der Diisononylphthalate kann z. B. an einem Katalysator, der mindestens ein Metall aus der VIII Nebengruppe, insbesondere aus der Triade Eisen, Kobalt, Nickel optional zusammen mit mindestens einem Metall der II, III, IV, V und/oder VI Nebengruppe des Periodensystems enthält, durchgeführt werden. Ebenso ist es möglich die Hydrierung der Diisononylphthalate an Ruthenium-haltigen Katalysatoren durchzuführen. Solche Verfahren sind z. B. in EP 1 042 273, EP 1 314 714 und EP 0 814 098 beschrieben. Als Katalysatoren können insbesondere solche eingesetzt werden, die auf einem Träger basieren, der einen Makroporenanteil von kleiner 5 % aufweist, wie z. B. Aerolyst 7711, Degussa AG. Solche Katalysatoren und die entsprechenden Hydrierungsverfahren sind z. B. in DE 102 25 565 und DE 102 32 868 beschrieben, auf deren Inhalt ausdrücklich Bezug genommen wird.

Das Verfahren zur Herstellung von Gemischen von Diisononylestern der 1,2-Cyclohexandicarbonsäure, zeichnet sich dadurch aus, dass bei der Herstellung der Diisononylester ein Gemisch isomerer Nonanole eingesetzt wird, welches einen Verzweigungsgrad von 1,2 bis 2,0 aufweist. Vorzugsweise werden in dem erfindungsgemäßen Verfahren Gemische isomerer Nonanole eingesetzt, die eine relativ geringe Verzweigung, vorzugsweise mit einen Verzweigungsgrad von 1,2 bis 1,9, bevorzugt einen Verzweigungsgrad von 1,3 bis 1,8 und besonders bevorzugt von 1,3 bis 1,7 aufweisen. Der Verzweigungsgrad gibt dabei die Anzahl der Verzweigungen im Molekül an. 1-Nonanol weist beispielsweise einen Verzweigungsgrad von 0 auf, 3,5,5-Trimethylhexanol weist einen Verzweigungsgrad von 3 auf. Der Verzweigungsgrad des Gemisches ergibt sich aus der Summe der Verzweigungsgrade der Einzelkomponenten multipliziert mit dem jeweiligen Anteil der Einzelkomponente geteilt durch die Summe der Anteile aller Einzelkomponenten. Der Verzweigungsgrad für Gemische kann im einfachsten Fall, durch direktes bestimmen der Anteile der Einzelkomponenten bestimmt werden. Ist eine solche Bestimmung nicht möglich, so kann der Verzweigungsgrad für Gemische primärer isomerer Nonanole z. B. mittels $^1$H-NMR analog zur oben beschriebenen Methode bestimmt werden. Da in einem $^1$H-NMR-Spektrum eines Gemisches von primären $C_9$-Alkoholen die Signale, die im Wesentlichen den Wasserstoffatomen der Methylengruppe, die dem Sauerstoff des Alkohols bzw. des Alkoholrests benachbart ist, zugeordnet werden, im Bereich von 3,0 bis 3,9 ppm auftreten, werden die Integralwerte der Signale im Bereich von 0,5 bis zum Minimum des niedrigsten Tals im Bereich von 0,9 bis 1,1 ppm und der Signale im Bereich von 3,0 bis 3,9 ppm bestimmt. Der Verzweigungsgrad V kann für ein Isomerengemisch wiederum gemäß der oben genannten Formel I berechnet werden.

Besonders bevorzugt enthalten die im erfindungsgemäßen Verfahren eingesetzten Gemische isomerer Nonanole weniger als 10 Mol-%, vorzugsweise weniger als 5 Mol-%, bevorzugt weniger als 1 Mol-% und insbesondere von 0 bis 0,5 Mol-%, bevorzugt weniger als 0,1 Mol-%, insbesondere von 0,0001 bis 0,1 Mol-% und besonders bevorzugt weniger als 0,05 Mol-%, insbesondere von 0,01 bis 0,05 Mol % an 3,5,5-Trimethylhexanol. Die Isomerenverteilungen in den Gemischen der isomeren Nonanole können mit den üblichen, dem Fachmann geläufigen Messmethoden wie NMR-Spektroskopie, GC- oder GC-MS-Spektroskopie ermittelt werden.

Die im erfindungsgemäßen Verfahren eingesetzten Gemische isomerer Nonanole können generell durch Hydroformylierung von Octenen, die wiederum auf unterschiedliche Art erzeugt werden können, hergestellt werden. Als Rohstoff zur Herstellung der Octene dienen im Allgemeinen technische $C_4$-Ströme, die zunächst alle isomeren $C_4$-Olefine neben den gesättigten Butanen und gegebenenfalls Verunreinigungen wie $C_3$- und $C_5$-Olefinen und acetylenischen Verbindungen enthalten. Durch Oligomerisierung dieses Olefingemisches erhält man vorwiegend isomere Octengemische neben höheren Oligomeren wie $C_{12}$- und $C_{16}$-Olefingemischen. Diese Octengemische werden zu den entsprechenden Aldehyden hydroformyliert und anschließend zum Alkohol hydriert. Die Zusammensetzung, d. h. die Isomerenverteilung der technischen Nonanolgemische ist abhängig vom Ausgangsmaterial und von den Oligomerisierungs- und Hydroformylierungsverfahren.

Als Octen-Gemische können z. B. auch solche eingesetzt werden, die über das sogenannte Polygas-Verfahren erhalten werden, bei dem eine Oligomerisierung von $C_3$-/$C_4$-Mischungen an einem festen sauren Katalysator, vorzugsweise an einem festen Phosphorsäure-Katalysator (SPA-Verfahren) durchgeführt wird. Dieses Verfahren wird unter anderem in den Dokumenten US 6,284,938, US 6,080,903, US 6,072,093, US 6,025,533, US 5,990,367, US 5,895,830, US 5,856,604, US 5,847,252 und US 5,081,086 beschrieben. Die nach diesen Verfahren erhaltenen Nonanole enthalten in der Regel auch noch Anteile von Octanolen und Decanolen, so dass hier die mittlere Kettenlänge von 9 Kohlenstoffatomen abweichen kann. Auf die Bestimmung des Verzweigungsgrads V gemäß der oben genannten Methode hat dies aber keine Auswirkung.

Besonders bevorzugte und im erfindungsgemäßen Verfahren einsetzbare Gemische isomerer Nonanole sind solche, die erhältlich sind durch Hydroformylierung und anschließende oder gleichzeitige Hydrierung eines Gemisches von isomeren Octenen, wobei das Gemisch isomerer Octene durch Inkontaktbringen eines Butene aufweisenden Kohlenwasserstoffgemisches, welches einen Anteil an Isobuten von vorzugsweise kleiner 20 Gew.-%, bevorzugt kleiner 10 Gew.-%, besonders bevorzugt kleiner 5 Gew.-%, ganz besonders bevorzugt kleiner 3 Gew.-%, insbesondere bevorzugt kleiner 1 Gew.-%, vorzugsweise zwischen 0,01 und 1 Gew.-% und besonders bevorzugt zwischen 0,05 und 0,5 Gew.-% bezogen auf die Butene aufweist, mit einem Oligomerisierungskatalysator, insbesondere mit einem Nickeloxid enthaltenden Katalysator, erhalten wird. Die Herstellung von isomeren Octenen durch Oligomerisierung von im wesentlichem linearen Butenen an Nickelträgerkatalysatoren ist z. B. als OCTOL-Prozess bekannt, der z. B. in EP 0 395 857 oder EP 1 029 839 beschrieben wird. In Varianten zum OCTOL-Prozess werden z. B. Ti oder Zr aufweisende Katalysatoren eingesetzt. Solche alternative Varianten und insbesondere die Katalysatoren werden z. B. in EP 1 171 413 beschrieben.

Die Gemische isomerer Octene werden anschließend einer Hydroformylierung zugeführt. Die Hydroformylierung kann in Gegenwart von modifizierten oder unmodifizierten Kobalt- oder Rhodiumkatalysatoren erfolgen. Vorzugsweise erfolgt die Hydroformylierung in Gegenwart von unmodifizierten Kobaltverbindungen. Der Hydroformylierung folgt anschließend üblicherweise eine Hydrierung. Solche Hydroformylierungs-/Hydrierungs-Verfahren sind z. B. aus EP 0 850 905 und EP 1 172 349 bekannt, auf die hier ausdrücklich verwiesen wird. Die Hydroformylierung kann auch in Gegenwart von Rhodiumkatalysatoren erfolgen. Solche Hydroformylierungsverfahren sind allgemein bekannt. Spezielle Verfahren zur Hydroformylierung, die zur Herstellung von im erfindungsgemäßen Verfahren einsetzbaren Gemischen isomerer Nonanole besonders gut geeignet sind, z. B. in WO 2004/020380 oder DE 103 27 435 beschrieben, auf die hier ausdrücklich verwiesen wird. Die dort beschriebenen Verfahren werden in Gegenwart von cyclischen Kohlensäureestern durchgeführt.

Es kann auch vorteilhaft sein, das Gemisch isomerer Octene vor der Zuführung zur Hydroformylierung zunächst wie in EP 1 172 349 beschrieben zu fraktionieren. Auf diese Weise ist es möglich, Octenfraktionen zu erhalten, die besonders gut zur Herstellung von im erfindungsgemäßen Verfahren einsetzbaren Gemischen isomerer Nonanole geeignet sind. Aus den Fraktionen kann dann auf relativ einfache Weise durch Mischen von geeigneten Fraktionen ein Gemisch von isomeren Octenen erhalten werden, welches zur Herstellung von Gemischen von isomeren Nonanolen zum Einsatz im erfindungsgemäßen Verfahren geeignet ist.

Im erfindungsgemäßen Verfahren kann aber auch als Gemisch isomerer Nonanole ein Gemisch eingesetzt werden, das durch Mischen von isomerenreinen Nonanolen und/oder Fraktionen von mehreren isomeren Nonanolen erhalten wird. Zahlreiche isomerenreine Nonanole sind kommerziell erhältlich. Ebenso sind Nonanol-Gemische oder -Fraktionen kommerziell erhältlich, die nicht die für das erfindungsgemäße Verfahren bevorzugten Eigenschaften aufweisen. Durch einfaches Mischen von solchen isomerenreinen Nonanolen mit Nonanol-Gemischen lassen sich Gemische von Nonanolen herstellen, die bei der Veresterung mit Cyclohexandisäure bzw. deren Anhydrid zu Estern mit den gewünschten Eigenschaften führen. Insbesondere ist es durch ein solches einfaches Mischen möglich, Gemische von Nonanolen zu erhalten, die den gewünschten Anteil an 3,5,5-Trimethylhexanol und an sonstigen Komponenten aufweisen.

In dem erfindungsgemäßen Verfahren zur Herstellung von Gemischen von Diisononylestern der 1,2-Cyclohexandicarbonsäure wird zunächst das Gemisch isomerer Nonanole, das einen Verzweigungsgrad von 1,2 bis 2,0, vorzugsweise einen Verzweigungsgrad von 1,2 bis 1,9, bevorzugt 1,3 bis 1,8 und besonders bevorzugt von 1,3 bis 1,7 aufweist, mit Phthalsäureanhydrid in einem Veresterungsschritt zu Diisononylphthalaten (DINP) umgesetzt und anschließend werden diese Diisononylphthalate hydriert (gemäß Anspruch 1).

Die Veresterung kann auf bekannte Weise z. B. durch Reaktion von Phthalsäureanhydrid mit einem geeigneten Gemisch isomerer Nonanole erfolgen. Prinzipiell sind alle bekannten Veresterungsverfahren in dem erfindungsgemäßen Verfahren als Veresterungsschritt einsetzbar. Vorzugsweise erfolgt der Veresterungsschritt allerdings nach einem Verfahren, bei dem das Reaktionswasser durch azeotrope Destillation mit dem Alkohol entfernt wird und 100 bis 110 % der durch die azeotrope Destillation aus der Reaktion entfernte Flüssigkeitsmenge mit dem Alkohol wieder ergänzt wird. Als Flüssigkeitsmenge wird im Folgenden das durch azeotrope Destillation aus der Reaktion entfernte Flüssigkeitsvolumen, hauptsächlich bestehend aus Reaktionswasser und Alkohol, bezeichnet.

Es kann auch erforderlich sein, mehr als die abdestillierte Flüssigkeitsmenge in den Reaktor zurückzuführen, d. h. neben der entfernten Alkoholmenge wird das Reaktionswasser ersetzt und darüber hinaus weiterer Alkohol zugegeben. In erfindungsgemäßen Verfahren zur Veresterung wird 110 bis 100 %, bevorzugt 105 bis 100 % der entfernten Flüssigkeitsmenge durch Alkohol ersetzt.

Diese Ausführungsform der Veresterung hat den Vorteil, dass im Vergleich zu bekannten diskontinuierlichen Verfahren die Reaktionsgeschwindigkeit erhöht wird. Dadurch kann die Taktzeit verkürzt werden, wodurch eine höhere Raum-Zeit-Ausbeute erreicht wind.

Die Veresterung kann autokatalysiert oder katalysiert durchgeführt werden. Als Veresterungskatalysatoren können Lewis- oder Bröndstedtsäuren oder metallorganische Stoffe, die nicht unbedingt als Säure wirken müssen, eingesetzt werden. Bevorzugte Veresterungskatalysatoren sind Alkoholate, Carbonsäuresalze oder Chelatverbindungen von Titan oder Zirkonium, wobei das Katalysatormolekül ein oder mehrere Metallatome enthalten kann. Insbesondere werden Tetra(isopropyl)orthotitanat und Tetra(butyl)orthotitanat eingesetzt.

Die Veresterung wird vorzugsweise in einem Reaktionsgefäß durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Ist ein Einsatzstoff bei der Einfülltemperatur fest, ist es zweckmäßig, die flüssige Einsatzkomponente vorzulegen. Feste Einsatzstoffe können als Pulver, Granulat, Kristallisat oder Schmelze eingespeist werden. Um die Chargenzeit zu verkürzen, ist es ratsam, während des Einfüllens mit dem Aufheizen zu beginnen. Der Katalysator kann in reiner Form oder als Lösung, bevorzugt gelöst in einem der Einsatzstoffe, zu Beginn oder erst nach Erreichen der Reaktionstemperatur eingebracht werden. Carbonsäureanhydride reagieren häufig mit Alkoholen bereits autokatalytisch, d. h. unkatalysiert zu den entsprechenden Estercarbonsäuren (Halbestern), beispielsweise Phthalsäureanhydrid zum Phthalsäuremonoester. Daher ist ein Katalysator häufig erst nach dem ersten Reaktionsschritt erforderlich.

Der umzusetzende Alkohol, der als Schleppmittel dient, kann im stöchiometrischen Überschuss eingesetzt werden. Bevorzugt wird ein Überschuss von 5 bis 50 %, besonders bevorzugt 10 bis 30 % eingesetzt.

Die Katalysatorkonzentration hängt von der Art des Katalysators ab. Bei den bevorzugt eingesetzten Titanverbindungen beträgt diese 0,005 bis 1,0 Massen-% bezogen auf das Reaktionsgemisch, insbesondere 0,01 bis 0,3 Massen-%.

Die Reaktionstemperaturen liegen bei Verwendung von Titankatalysatoren zwischen 160 °C und 270 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei verminderten Druck durchgeführt. Beispielsweise wird bei der Umsetzung von Phthalsäureanhydrid mit einem Gemisch isomerer Nonanole in einem Temperaturbereich von 170 °C bis 250 °C im Druckbereich von 0,1 MPa bis 1,0 kPa gearbeitet.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem Vorratsgefäß bereitstehenden Alkohol zu ersetzen. In anderen Ausführungsformen der Veresterung wird die abgetrennte Flüssigkeit zum Alkohol, vorzugsweise zum reinen Alkohol aufgearbeitet.

Nach Beendigung der Reaktion enthält das Reaktionsgemisch, das im Wesentlichen aus Vollester (Zielprodukt) und überschüssigem Alkohol besteht, neben dem Katalysator und/oder dessen Folgeprodukte geringe Mengen an Estercarbonsäure(n) und/oder nicht umgesetzter Carbonsäure. Zur Aufarbeitung dieser Esterrohgemische wird der überschüssige Alkohol entfernt, die sauren Verbindungen neutralisiert, der Katalysator zerstört und die dabei entstandenen festen Nebenprodukte abgetrennt. Dabei wird der größte Teil des Alkohols bei Normaldruck oder im Vakuum abdestilliert. Die letzten Spuren des Alkohols können z. B. durch Wasserdampfdestillation, insbesondere im Temperaturbereich von 120 bis 225 °C, entfernt werden. Die Abtrennung des Alkohols kann beispielsweise als erster oder als letzter Aufarbeitungsschritt erfolgen.

Die Neutralisation der sauren Stoffe, wie Carbonsäuren, Estercarbonsäuren oder gegebenenfalls der sauren Katalysatoren, kann durch Zugabe von basisch wirkenden Verbindungen der Alkali- und Erdalkalimetalle erfolgen. Diese können in Form ihrer Carbonate, Hydrogencarbonate oder Hydroxide eingesetzt werden. Das Neutralisationsmittel kann in fester Form oder bevorzugt als Lösung, insbesondere als wässrige Lösung eingesetzt werden. Die Neutralisation kann sofort nach Beendigung der Veresterungsreaktion oder nach Abdestillation der Hauptmenge des überschüssigen Alkohols durchgeführt werden. Bevorzugt ist die Neutralisation mit Natronlauge sofort nach Beendigung der Veresterungsreaktion bei Temperaturen über 150 °C. Das mit der Lauge eingebrachte Wasser kann dann zusammen mit Alkohol abdestilliert werden.

Weitere Details zu geeigneten Veresterungsverfahren, die in dem erfindungsgemäßen Verfahren als Veresterungsschritt eingesetzt werden können, können z. B. EP 1 186 593 und EP 1 300 388, auf die ausdrücklich verwiesen wird, entnommen werden.

Es kann besonders vorteilhaft sein, wenn die Veresterung so durchgeführt wird, wie sie in DE 10 2005 021 075.9, auf die ausdrücklich Bezug genommen wird, beschrieben wird. Das dort beschriebene Verfahren zur Herstellung von Carbonsäureestern durch metallkatalysierte Reaktion von Mono-, Di- oder Polycarbonsäuren oder deren Anhydriden mit Alkohol in Gegenwart eines Überschusses an Alkohol, wobei der überschüssige Alkohol nach der Veresterung entfernt, der so erhaltene Rohester durch Basenzugabe neutralisiert und anschließend filtriert wird, und wobei zumindest ein Teil des überschüssigen Alkohols durch mindestens eine Wasserdampfdestillation entfernt wird, zeichnet sich dadurch aus, dass

a) in einem ersten Schritt, vorzugsweise nach Beendigung der Veresterungsreaktion, durch Destillation der Alkoholgehalt im Veresterungsgemisch auf einen Anteil von kleiner-gleich 5 Massen-% reduziert wird,
b) dem in Schritt a) erhaltenen Rohester eine erste Menge Base zugesetzt wird, so dass die Base, in Basenäquivalenten gerechnet, mit dem Metallatom des eingesetzten Veresterungskatalysator im molaren Verhältnis von 10 zu 1 bis 1 zu 1 steht,
c) das in Schritt b) erhaltene Gemisch einer Wasserdampfdestillation unterzogen wird und dass zu Beginn und/oder im Verlauf der Wasserdampfdestillation eine zweite Menge Base dem Gemisch zugegeben wird, die mindestens der Menge entspricht, die zur Neutralisation von Restsäure notwendig ist.

Die Hydrierung der Diisononylphthalate kann in dieser Ausführungsform des erfindungsgemäßen Verfahrens z. B. an einem Katalysator, der mindestens ein Metall der VIII Nebengruppe, insbesondere mindestens ein Metall aus der Triade Eisen, Kobalt, Nickel optional zusammen mit mindestens einem Metall der II, III, IV, V und/oder VI Nebengruppe des Periodensystems enthält, durchgeführt werden. Bevorzugte Metalle der II, III, IV, V, und/oder VI Nebengruppe sind Zink und/oder Chrom. Ganz besonders bevorzugt ist es möglich die Hydrierung der Diisononylphthalate an Ruthenium-haltigen Katalysatoren durchzuführen. Solche Katalysatoren sind z. B. in EP 1 042 273, EP 1 314 714 und EP 0 814 098 beschrieben, auf welche ausdrücklich verwiesen wird. Als Katalysatoren werden aber bevorzugt solche eingesetzt, die einen Träger aufweisen, der einen Makroporenanteil von kleiner als 5 % aufweist. Solche Katalysatoren werden z. B. in DE 102 25 565 und DE 102 32 868 beschrieben, auf die ausdrücklich Bezug genommen wird.

Als Katalysatoren für die Hydrierung können weiterhin die in WO 2004/046078, auf die ausdrücklich Bezug genommen wird, beschriebenen Katalysatoren eingesetzt werden. Diese Katalysatoren weisen ein hydrieraktives Metall, vorzugsweise ein Übergangsmetall der Gruppe VIII, insbesondere ausgewählt aus der Gruppe umfassend Platin, Rhodium, Palladium, Kobalt, Nickel oder Ruthenium oder aus der I oder VII Übergangsgruppe des Periodensystem der Elemente, auf einem Trägermaterial auf, welches ein geordnete Mesoporen aufweisendes Material aufweist. Als geordnete Mesoporen aufweisendes Material kann z. B. Kieselsäure eingesetzt werden.

Darüber hinaus können alle im erfindungsgemäßen Verfahren eingesetzten Katalysatoren zusätzlich eine inerte Komponente (Träger), die mindestens ein Metall aus der Gruppe Al, Mg, Ti, Zr und/oder Si, als Oxid oder Mischoxid enthält, aufweisen. Optional können die Katalysatoren auch Salze der oben genannten Metalle, wie beispielsweise Sulfate und/oder Phosphate enthalten. Weiterhin können die erfindungsgemäß eingesetzten Katalysatoren Verarbeitungs- und Formgebungshilfsmittel wie beispielsweise Graphit beinhalten.

Nachfolgend werden bevorzugte Zusammensetzungen angegeben. Die Zusammensetzungen beziehen sich jeweils auf die reduzierten Katalysatoren.

Der Gehalt der Katalysatoren an den genannten Metallen der VIII Nebengruppe (berechnet als Metall) liegt vorzugsweise im Bereich von 1 bis 60 Massen-%, insbesondere im Bereich von 25 bis 45 Massen-%, ganz besonders bevorzugt im Bereich von 30 bis 40 Massen-%.

Der Gehalt der Katalysatoren an Metallen der II, III, IV, V und/oder VI Nebengruppe (berechnet als Oxid) beträgt 10 bis 90 Massen-%, insbesondere 20 bis 60 Massen-%, ganz besonders 20 bis 40 Massen-%.

In der Hydrierung werden besonders bevorzugt Katalysatoren eingesetzt, die in reduzierter, aktiver Form Nickel zumindest teilweise in der Oxidationsstufe 0 und Zink vorzugsweise in der Oxidationsstufe +2 enthalten.

Die Katalysatoren werden nach an sich bekannten Verfahren hergestellt. Vorzugsweise werden die Katalysatoren durch Ausfällen von löslichen Metallsalzen hergestellt. Um einen Katalysator herzustellen, der beispielsweise die Hauptkomponenten Nickel, Zinkoxid und als Träger Siliziumdioxid enthält, können beispielsweise Nickel- und Zinkcarbonat in einer Suspension von Kieselsäure und gegebenenfalls Graphit in Wasser ausgefällt werden. Weitere, dem Fachmann bekannte Schritte zur Herstellung des Katalysators sind: Abtrennung und Wäsche des Niederschlags, Trocknung, Calzinierung, Formgebung und Reduktion.

Die Katalysatoren werden zweckmäßig in eine Form gebracht, die bei der Hydrierung einen geringen Strömungswiderstand bietet, wie beispielsweise Tabletten, Zylinder, Strangextrudate oder Ringe.

In dieser Ausführungsform des erfindungsgemäßen Verfahrens kann im Schritt der Hydrierung beispielsweise der käuflich verfügbare Katalysator H10126 der Degussa AG, Düsseldorf, eingesetzt werden. Dieser Katalysator wird bislang nur für die Hydrierung von aromatischen und olefinischen Kohlenwasserstoffen in Halogen- und Schwefel enthaltenden Rohstoffen eingesetzt. Sein Einsatz für die Kernhydrierung von aromatischen Estern ist nur selten beschrieben worden. Dieser Katalysator enthält 32 Massen-% Nickel, 29 Massen-% Zinkoxid, 24 Massen-% Siliziumdioxid.

Neben Nickel-aufweisenden Katalysatoren können insbesondere auch Katalysatoren eingesetzt werden, die Ruthenium als Aktivmetall aufweisen. Neben Ruthenium kann zusätzlich mindestens ein Metall der ersten und/oder siebten Nebengruppe des Periodensystems der Elemente in den Katalysatoren als Aktivmetall enthalten sein. Bevorzugt wird als weiteres Aktivmetall Rhenium und/oder Kupfer eingesetzt.

Bevorzugt sind die eingesetzten Ruthenium-aufweisenden Katalysatoren Trägerkatalysatoren. Als Träger können beispielsweise folgende Stoffe verwendet werden: Aktivkohle, Siliciumcarbid, Aluminiumoxid, Siliciumoxid, Alumosilikat, Titandioxid, Zirkoniumdioxid, Magnesiumoxid und/oder Zinkoxid oder deren Gemische. Besonders bevorzugt wird ein Katalysator eingesetzt, der einen Titandioxid-Träger aufweist. Zusätzlich können diese Trägermaterialien Alkalimetalle, Erdalkalimetalle und/oder Schwefel enthalten.

Der Gehalt der Aktivmetalle, d. h. der Metalle der ersten und/oder siebten und/oder achten Nebengruppe des Periodensystems der Elemente im Katalysator beträgt im Allgemeinen 0,1 bis 30 Massen-%. Der Rutheniumgehalt, berechnet als Metall liegt vorzugsweise im Bereich von 0,1 bis 10 Massen-%, insbesondere im Bereich von 0,8 bis 5 Massen-%, ganz besonders im Bereich zwischen 1 und 3 Massen-%.

Die Herstellung solcher Ruthenium-haltigen Katalysatoren kann den Dokumenten DE 102 25 565 und DE 102 32 868 entnommen werden, auf die ausdrücklich verwiesen wird.

Die Hydrierung in dieser Ausführungsform des erfindungsgemäßen Verfahrens wird bevorzugt in flüssiger Phase durchgeführt. Die Hydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Hydrierung an einem im Festbett angeordnetem Katalysator, bei dem sich unter Reaktionsbedingungen die Produkt/Eduktphase hauptsächlich im flüssigen Zustand befindet, bevorzugt.

Wenn die Hydrierung kontinuierlich an einem im Festbett angeordneten Katalysator durchgeführt wird, ist es zweckmäßig, den Katalysator vor der Hydrierung in die aktive Form zu überführen. Dies kann durch Reduktion des Katalysators mit Wasserstoff-haltigen Gasen nach einem Temperaturprogramm erfolgen. Dabei kann die Reduktion gegebenenfalls in Gegenwart einer flüssigen Phase, die über den Katalysator rieselt, durchgeführt werden. Als flüssige Phase kann dabei ein Lösemittel oder das Hydrierprodukt eingesetzt werden.

Für die Hydrierung können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Fall kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die aromatischen Polycarbonsäureester im geraden Durchgang oder unter Produktrückführung zu hydrieren.

Die Hydrierung kann in der Flüssig/Gas-Mischphase oder in der Flüssigphase in Dreiphasenreaktoren im Gleichstrom durchgeführt werden, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120, insbesondere von 25 bis 80 $m^3$ pro $m^2$ Querschnitt des leeren Reaktors und Stunde betrieben. Wird ein Reaktor im geraden Durchgang betrieben, so kann die spezifische Katalysatorbelastung (LHSV) Werte zwischen 0,1 und 10 $h^{-1}$ annehmen.

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Beispielsweise können folgende Stoffe als Lösemittel eingesetzt werden:
Geradkettige oder cyclische Ether, wie beispielsweise Tetrahydrofuran oder Dioxan sowie aliphatische Alkohole, in denen der Alkylrest 1 bis 13 Kohlenstoffatome aufweist. Bevorzugt verwendbare Alkohole sind beispielsweise Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde, also hier das Gemisch von isomeren Nonanolen. Dadurch ist die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zu Folge haben. Bevorzugt liegt der Aromatengehalt im Reaktorzulauf zwischen 1 und 35 %, insbesondere zwischen 5 und 25 %. Der gewünschte Konzentrationsbereich kann bei Reaktoren, die in Schlaufenfahrweise betrieben werden, durch die Zirkulationsrate (Mengenverhältnis von rückgeführten Hydrieraustrag zu Edukt) eingestellt werden.

Die Hydrierung kann in einem Druckbereich von 3 bis 25 MPa, insbesondere von 5 bis 10 MPa durchgeführt werden. Die Hydriertemperaturen liegen vorzugsweise im Bereich von 60 bis 200°C, insbesondere im Bereich von 80 bis 140 °C.

Als Hydriergase können beliebige Wasserstoff-haltige Gasgemische, die keine schädlichen Mengen an Katalysatorgiften wie beispielsweise Kohlenmonoxid oder Schwefelwasserstoff enthalten, eingesetzt werden. Die Inertgasbestandteile können beispielsweise Stickstoff oder Methan sein. Bevorzugt wird Wasserstoff in einer Reinheit von größer 95 %, insbesondere größer 98 % eingesetzt.

Die hergestellten Gemische von Diisononylestern der 1,2-Cyclohexandicarbonsäure können in Farben, Tinten oder Lacken, in Plastisolen, Klebstoffen oder Klebstoffkomponenten, in Dichtungsmassen oder als Weichmacher in Kunststoffen oder Kunststoffkomponenten oder als Lösemittel verwendet werden. Bevorzugte Plastisole, sind insbesondere PVC-Plastisole. Bevorzugte Kunststoffe sind PVC, Homo- und Copolymere auf Basis von Ethylen, Propylen, Butadien, Vinylacetat, Glycidylacrylat, Glycidylmethacrylat, Methacrylaten, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.

Als Vertreter der obigen Gruppen seien beispielsweise folgende Kunststoffe genannt: Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, Polymethacrylat, Polymethylmethacrylat, Methylacrylat-Butylacrylat-Copolymere, Methylmethacrylat-Butylmethacrylat-Copolymere, Ethylen-Vinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere, PVB und PVC.

Darüber hinaus können die hergestellten Gemische zur Modifizierung von Kunststoffmischungen, beispielsweise der Mischung eines Polyolefins mit einem Polyamid, eingesetzt werden.

Gemische aus Kunststoffen, insbesondere PVC, die hergestellte Gemische von Diisononylestern der 1,2-Cyclohexandicarbonsäure enthalten, können beispielsweise in folgenden Produkten enthalten sein:
Gehäuse für Elektrogeräte, wie beispielsweise Küchengeräte, Computergehäuse, Gehäuse und Bauteile von Phono- und Fernsehgeräte, Rohrleitungen, Apparaten, Kabeln, Drahtummantelungen, Isolierbändern, im Innenausbau, im Fahrzeug- und Möbelbau, Plastisolen, in Bodenbelägen, medizinische Artikel, Lebensmittelverpackungen, Dichtungen, Folien, Verbundfolien, Schallplatten, Kunstleder, Spielzeug, Verpackungsbehälter, Klebebandfolien, Bekleidung, Beschichtungen, Fasern für Gewebe, beschichtete Gewebe. Weiterhin können Gemische aus Kunststoff, insbesondere PVC, die hergestellte Gemische von Diisononylestern der 1,2-Cyclohexandicarbonsäure enthalten, beispielsweise zur Herstellung folgender Erzeugnisse verwendet werden:
Gehäuse für Elektrogeräte, Rohrleitungen, Schläuchen, Kabel, Draht-Ummantelungen, Isolierbändern, im Fahrzeug- und Möbelbau, Plastisolen, Fensterprofile, Bodenbeläge, medizinische Artikel (wie z. B. Blutbeutel), Spielzeuge, Lebensmittelverpackungen, Dichtungen, Folien, Verbundfolien, Platten, Schallplatten, Kunstleder, Tapeten, Verpackungsbehälter, Klebebandfolien, Bekleidung, Beschichtungen oder Fasern für Gewebe, Schuhe, Unterbodenschutz, Nahtabdichtungen, Modelliermassen oder Bällen.

Solche Gemische aus Kunststoff, insbesondere Weich-PVC oder Plastisole, die PVC und hergestellte Gemische von Diisononylestern der 1,2-Cyclohexandicarbonsäure enthalten, enthalten vorzugsweise von 5 bis 120 Massenteile, bevorzugt von 10 bis 100 Massenteile und besonders bevorzugt von 20 bis 80 Massenteile an den hergestellten Gemischen von Diisononylestern der 1,2-Cyclohexandicarbonsäure pro 100 Massenteilen PVC.

Neben den obengenannten Anwendungen können die hergestellten Gemische von Diisononylestern der 1,2-Cyclohexandicarbonsäure als Schmierölkomponente, als Bestandteil von Kühlflüssigkeiten und Metallbearbeitungsflüssigkeiten verwendet werden.

Die folgenden Beispiele sollen die Erfindung erläutern, ohne den Schutzbereich, definiert durch die Patentansprüche, einzuschränken.

**Beispiele** (nicht erfindungsgemäß)

Beispiel 1: Herstellung von 3,5,5-Trimethylhexanol

In einem 2 1 Autoklav wurden 1000 g 2,4,4-Trimethyl-1-penten (Diisobuten, Hersteller OXENO Olefinchemie GmbH) bei 135 °C unter 270 bar Synthesegasdruck 3 Stunden lang in Gegenwart eines unmodifzierten Rhodium-Katalysators hydroformyliert. Der aktive Katalysator wurde in situ aus Rhodium-Nonanoat (mit 24,8 Gew.-% Rh) generiert. Die Rhodiumkonzentration bezogen auf Diisobuten wurde auf 20 ppm eingestellt. Nach 3 Stunden wurde die Reaktion abgebrochen und der Autoklav auf 20 °C abgekühlt. Der Reaktionsaustrag enthielt 93,5 Gew.-% 3,5,5-Trimethylhexanal, 2,5 Gew.-% 3,5,5-Trimethylhexanol, 3,4 Gew.-% $C_8$-Restkohlenwasserstoffe und 0,6 Gew.-% Hochsieder (bestimmt mit GC). In einer Labordestillationskolonne wurde der Reaktionsaustrag durch Destillation vom RhodiumKatalysator befreit.

Der Rh-freie Hydroformylierungsaustrag wurde anschließend in der flüssigen Phase in einem Festbettreaktor in Gegenwart eines Cu/Cr/Ni-Katalysators (H14279; Degussa AG, Düsseldorf) bei 180 °C und 25 bar hydriert. Nach der Hydrierung von 3,5,5-Trimethylhexanal zu dem Zielprodukt 3,5,5-Trimethylhexanol wurde der Hydrieraustrag durch gezielte Destillation von den Leichtsiedern (C$_8$-Kohlenwasserstoffe) befreit. Nach der Destillation wurde ein 3,5,5-Trimethylhexanol mit einer Reinheit von über 99,5 Gew.-% erhalten.

Beispiel 2: Herstellung verschiedener Cyclohexan-1.2-dicarbonsäurediisononylester (DINCH)

Die in Tabelle 1 aufgeführten, bis auf A kommerziell erhältlichen C$_9$-Alkohole wurden nach folgender Vorschrift zu den entsprechenden Cyclohexan-1,2-dicarbonsäureestern umgesetzt: In einem 2 1 Destillationskolben wurden 462 g (3 Mol) cis-Hexahydrophthalsäureanhydrid (Fluka,), 1296 g Nonyl-Alkohol (9 Mol) entsprechend Tabelle 1, 200 ml Toluol und 1,94 g Tetra-iso-Nonyltitanat vorgelegt und 7,5 Stunden bei 180 °C, mit Rückfluss, unter Normaldruck verestert. Die Temperatur wurde über die Zugabe von Toluol konstant gehalten. Nach 7,5 Stunden war die Säurezahl ≤ 0,1 mg KOH/g (hier und in den nachfolgend aufgeführten Beispielen bestimmt gemäß DIN EN ISO 2114, nach dem kolorimetrischen Titrationsverfahren gemäß Verfahren A, wobei als Lösemittel ein Toluol/Isopropanol/WasserGemisch mit einem Volumenverhältnis von 1 zu 1,5 zu 0,2 eingesetzt wurde), was einem über 99,9 % igen Umsatz des Cyclohexansäureanhydrids entspricht.

Im Anschluss wurden erst das Toluol, bis 120 °C bei 50 hPa und dann der Alkoholüberschuss, bis 180 °C bei 5 hPa über eine Claisenbrücke, abdestilliert. Über eine weitere Säurezahlbestimmung wurde die für die Neutralisation benötigte Natronlaugemenge ermittelt. Anschließend wurde in einem 2 1 Reaktionskolben der Ansatz bei 80 °C unter Normaldruck durch 30 Minuten Rühren mit Natronlauge neutralisiert.

Im Anschluss an die Neutralisation wurde die Apparatur evakuiert und auf 180 °C aufgeheizt. Über ein Tauchrohr mit aufgesetztem Tropftrichter wurde bei 180 °C und 5-20 hPa langsam Wasser zugetropft, um den Ester zu reinigen. Nach abgeschlossener Reinigung wurde die Heizung abgestellt und das Produkt unter Vakuum abgekühlt. Bei 100 °C wurde der Ester über eine Nutsche mit Filterpapier (Sorte 389, Filtrak) und Filterhilfsmittel (Perlite) gefiltert.

**Tabelle 1:**

| Bezeichnung | Herkunft / Handelsname |
|---|---|
| A | 3,5,5-Trimethylhexanol gem. Beispiel 1 (Vergleichsbeispiel) |
| B | EXXAL 9 (Exxon-Mobil)) |
| C | EXXAL 9-S (Exxon-Mobil) |
| D | Isononanol (OXENO) |
| E | Nonanol N (BASF) |

Beispiel 3: NMR-spektroskopische Untersuchung der verschiedenen DINCH-Typen

| | |
|---|---|
| Messgerät: | NMR-Spektrometer Avance 500 der Firma Bruker |
| Messfrequenz: | 500 MHz |
| Probenkopf: | BBO-Probenkopf, 5mm |
| Lösungsmittel: | CDCl$_3$ (Deuterierungsgrad 99,8 %) |
| Standard: | Tetramethylsilan (TMS) |
| Messtemperatur: | 300 K |
| Anzahl Scans (Durchgänge): | 32 |
| Delay (Verzögerung): | 5 s |
| Aquisitionszeit: | 3,3 s |
| Spektrale Breite: | 10000 Hz |
| Pulswinkel: | 30° |
| Pulslänge (90°): | 9,7 μs |

Zur Aufnahme der $^1$H-NMR-Spektren wurden ca. 20 mg der Probe in ca. 0,6 ml CDCl$_3$ (mit 1 Gew.-% TMS) gelöst und in ein NMR-Röhrchen mit einem Durchmesser von 5 mm gefüllt. Die Spektren wurden unter den oben angegebenen Bedingungen aufgenommen und auf TMS = 0 ppm referenziert. Die Fläche unter den Signalen im Bereich von 0,5 ppm bis zum Minimum des niedrigsten Tals im Bereich von 0,9 bis 1,1 ppm wurde integriert. Die Fläche in diesem Bereich wird im Wesentlichen bestimmt durch die Signale der Wasserstoffatome, die zu einer Methylgruppe gehören. Die Fläche unter den Signalen im Bereich von 3,6 ppm bis 4,4 ppm wurde ebenfalls integriert. Die Fläche in diesem Bereich wird im Wesentlichen bestimmt durch die Signale der Methylenwasserstoffatome der Methylengruppe, die dem Sauerstoff des Alkohols bzw. des Alkoholrestes benachbart ist. Die Integration erfolgte mit der Software xwinnmr 3.5 (Firma Bruker). Der Verzweigungsgrad V kann dann bestimmt werden aus dem Verhältnis des Flächenintegrals, welches im Wesentlichen den Methylwasserstoffatomen zugeordnet ist $I(CH_3)$, geteilt durch drei (für die drei Methylwasserstoffatome), zu dem Flächenintegral der Methylenwasserstoffatome in Nachbar-schaft zum Sauerstoff $I(OCH_2)$, geteilt durch 2 (für die zwei Methylenwasserstoffatome) minus eins gemäß folgender Formel:

$$V = 2/3 * I(CH_3)/I(OCH_2) - 1$$

Die Verzweigungsgrade der DINCH-Typen A bis E sind in Tabelle 2, Spalte 3 aufgelistet.

Beispiel 4: Bestimmung der Glasübergangstemperatur ($T_G$)

Mittels DSC-Messung nach DIN 51 007 (Ausgabe Juni 1994) wurden nun von den gemäß Beispiel 2 hergestellten Weichmachern und von DEHP die Glasübergangstemperatur bestimmt. Dazu wurden von 10 bis 15 mg Probe in einem dicht verschlossenen Aluminium-Tiegel (40 µl) mit einem DSC-Gerät der Firma Mettler, Typ DSC820 vermessen. Die Heizraten von -150 °C bis +50 °C betrugen 5 K/min. Die Abkühlung auf Starttemperatur erfolgte ungeregelt (schnellstmöglich). Da der Tiegel dicht verschlossen wurde, konnte ohne eine besondere Atmosphäre oder einen besonderen Gasfluss bei Umgebungsatmosphäre gearbeitet werden. Die Kalibrierung erfolgte mit Indium. Die Auswertung erfolgte mit der Software STARe 8.10. Die Ergebnisse sind in der folgenden Tabelle 2 aufgelistet

**Tabelle 2**

| Bezeichnung | Verwendeter Alkohol | Verzweigungsgrad gem. Beispiel 3 | Glasübergangstemperatur $T_G$ in °C gem. Beispiel 4 |
|---|---|---|---|
| DINCH-A | 3,5,5-Trimethylhexanol (gem. Beispiel 1, Vergleichsbeispiel) | 3,1 | -69,7 |
| DINCH-B | EXXAL 9 (ExxonMobil) | 1,91 | -83,2 |
| DINCH-C | EXXAL 9-S (ExxonMobil) | 1,72 | -85,4 |
| DINCH-D | Isononanol (OXENO) | 1,31 | -90,4 |
| DINCH-E | Nonanol N (BASF) | 1,24 | -91,0 |

Für DEHP wird nach dieser Methode eine Glasübergangstemperatur $T_G$ von -85,5 °C erhalten. Auf Grund der etwas geringeren weichmachenden Wirkung von DINCH gegenüber DEHP und einer damit gegebenenfalls notwendigen Höherdosierung von DINCH kann man jedoch davon ausgehen, dass auch DINCH-B noch prinzipiell geeignet wäre, welches in seinem $T_G$ nur wenig von dem des DEHP abweicht.

Verzweigungsgrad und Glasübergangstemperatur korrelieren in diesen Beispielen eindeutig. Mit der Funktion "KORREL" aus MS Excel kann hier ein Korrelationskoeffizient von > 0,99 erhalten werden.

Somit zeigt sich, dass bei Verzweigungsgraden oberhalb von 2 nicht mehr die notwendige Kälteflexibilität erzielt werden kann, die beispielweise bei der Lagerung von Medizinprodukten in Behältern aus weichgemachtem PVC notwendig ist.

Beispiel 5: Herstellung von Weich-PVC-Prüfkörpern

600 g Suspensions-PVC vom Typ Solvic 271 PC wurden mit 400 g Weichmacher gemäß Tabelle 2 sowie 24 g des Stabilisators BP MC 8823 (Fa. Baerlocher) mit einem Handmixer bei Raumtemperatur gemischt. Die Mischung wurde anschließend auf einem dampfbeheizten Labormischwalzwerk (Fa. Collin, Typ "150") plastifiziert und zu einem Walzfell verarbeitet. Die Temperatur der beiden Walzen betrug im Falle der Cyclohexandicarbonsäureester jeweils 170 °C, bei Verwendung des Weichmachers DEHP 165 °C. Die Walzzeit betrug 5 Minuten. Das abgekühlte Walzfell wurde dann in einer hydraulischen Hand-Presse (60 t) der Firma Werner & Pfleiderer wie folgt verpresst: Die Temperatur wurde auf 175 °C (beim DEHP haltigen Walzfell auf 170 °C) eingestellt und das Fell zunächst zwei Minuten bei 50 bar, dann eine Minute bei 100 bar und schließlich nochmals zwei Minuten bei 180 bar verpresst. Danach wurde der Druck auf 200 bar erhöht und hierbei auf Raumtemperatur abgekühlt.

Beispiel 6: Bestimmung der Glasübergangstemperatur der Prüfkörper

Aus den nach Beispiel 5 hergestellten Prüfkörpern wurden 60 mm lange, 8 mm breite und 1 mm dicke Stücke ausgestanzt und von diesen in einem Torsionspendel vom Typ MYRENNE ATM III nach DIN EN ISO 6721 (Teil 2, Verfahren B, Einspannlänge 50 mm) bei Temperaturen von -100 °C bis +100 °C und einer Frequenz von 1 s$^{-1}$ jeweils die Steifigkeit G' und der Verlustmodul G" bestimmt. Aus dem Maximum von G" ließ sich die Glasübergangstemperatur $T_G$ bestimmen. Diese ist ein Maß für die Flexibilität bei tiefen Temperaturen. Die Glasübergangstemperaturen der Prüfkörper sind in Tabelle 3 aufgelistet:

Beispiel 7: Bestimmung des Massenverlustes der Prüfkörper nach Wärmealterung

7 Tage nach der Herstellung der 1 mm dicken Pressplatten gemäß Beispiel 5 wurden hieraus Zugstäbe gemäß ISO 527 Typ 5 ausgestanzt und an der kurzen Seite gelocht. Danach wurden die Prüfkörper mindestens 24 h im Normklima (23 °C, 50 % relative Luftfeuchtigkeit) gelagert. Sodann wurden von jeder Serie drei Prüfkörper auf 0,1 mg genau auf einer Analysenwaage gewogen und dann hängend in einem Trockenschrank Heraeus (jetzt Kendro) UT 6060 sieben Tage bei 100 °C unter Frischluftzufuhr (ca. 2,6 m$^3$ pro Stunde) gelagert. Die Messung der Temperatur erfolgte über einen vom Gerät unabhängigen PT100 Temperaturfühler (im Innenraum montiert, Wandabstand min. 100 mm). Zur Einhaltung eines ausreichenden Abstandes zwischen den Proben (5 mm) wurden Raschigringe verwendet. Zu den Innenwänden wurde ein Mindestabstand von 30 mm eingehalten.
Nach der Alterung werden die Proben dem Trockenschrank entnommen und wiederum mindestens 24 h im Normklima (23 °C, 50 % relative Luftfeuchtigkeit) temperiert und anschließend wieder ausgewogen. Aus der Differenz der Einwaagen bezogen auf die Ausgangsmasse errechnet sich der prozentuale Massenverlust. Dieser ist in Tabelle 3 aufgeführt.

**Tabelle 3**

| Verwendeter Weichmacher | $T_G$ in °C Gem. Beispiel 7 | Masseverlust nach Wärmealterung (7d/ 100 °C) Gemäß Beispiel 8 |
|---|---|---|
| DINCH-A | -27 °C | 19 % |
| DINCH-D | -44 °C | 3 % |
| DEHP | -35 °C | 6 % |

Die Ergebnisse aus Tabelle 3 zeigen eindeutig eine Abhängigkeit der anwendungstechnischen Eigenschaften von der Struktur bzw. des Verzweigungsgrades des verwendeten DINCH. Es ist deutlich zu erkennen, dass mit einem DINCH auf Basis von 3,5,5-Trimethylhexanol deutlich schlechtere Eigenschaften erzielt werden als mit einem DINCH, das auf einem Alkohol basiert, der einen Verzweigungsgrad im beanspruchten Bereich aufweist.

**Patentansprüche**

1.   Verfahren zur Herstellung von Gemischen von Diisononylestern der 1,2-Cyclohexandicarbonsäure umfassend die Verfahrensschritte:

a) Umsetzung eines Gemisches isomerer Nonanole, das einen Verzweigungsgrad von 1,2 bis 2,0 aufweist, mit Phthalsäureanhydrid in einem Veresterungsschritt zu Diisononylphthalaten, wobei 110 bis 100 % der in der Reaktion abdestillierten Flüssigkeitsmenge durch Alkohol ersetzt wird,
b) Hydrierung dieser Diisononylphthalate.

2.   Verfahren nach Anspruch 1,
wobei der Verzweigungsgrad im Bereich von 1,2 bis 1,9 liegt.

3.   Verfahren nach einem der Ansprüche 1 oder 2,

wobei der Verzweigungsgrad im Bereich von 1,3 bis 1,8 liegt.

4. Verfahren nach einem der Ansprüche 1 bis 3,
wobei der Verfahrensschritt a) bei einer Temperatur erfolgt, welche im Bereich von 170 °C bis 250°C liegt und bei einem Druck erfolgt, welcher im Bereich von 0,1 MPa bis 1,0 kPa liegt.


**Claims**

1. Process for preparing mixtures of diisononyl esters of 1,2-cyclohexanedicarboxylic acid comprising the process steps of:

   a) reacting a mixture of isomeric nonanols which has a degree of branching of from 1.2 to 2.0 with phthalic anhydride in an esterification step to give diisononyl phthalates, wherein 110 to 100% of the amount of liquid distilled off in the reaction is replaced by alcohol,
   b) hydrogenating said diisononyl phthalates.

2. Process according to Claim 1,
wherein the degree of branching is in the range from 1.2 to 1.9.

3. Process according to one of Claims 1 and 2,
wherein the degree of branching is in the range from 1.3 to 1.8.

4. Process according to one of Claims 1 to 3,
wherein process step a) is effected at a temperature in the range from 170°C to 250°C and at a pressure in the range from 0.1 MPa to 1.0 kPa.


**Revendications**

1. Procédé de fabrication de mélanges d'esters diisononyliques de l'acide 1,2-cyclohexane-dicarboxylique, comprenant les étapes de procédé suivantes :

   a) la mise en réaction d'un mélange de nonanols isomères, qui présente un degré de ramification de 1,2 à 2,0, avec de l'anhydride de l'acide phtalique dans une étape d'estérification en phtalates de diisononyle, 110 à 100 % de la quantité de liquide distillée pendant la réaction étant remplacée par un alcool,
   b) l'hydrogénation de ces phtalates de diisononyle.

2. Procédé selon la revendication 1, dans lequel le degré de ramification se situe dans la plage allant de 1,2 à 1,9.

3. Procédé selon l'une quelconque des revendications 1 ou 2, dans lequel le degré de ramification se situe dans la plage allant de 1,3 à 1,8.

4. Procédé selon l'une quelconque des revendications 1 à 3, dans lequel l'étape de procédé a) a lieu à une température qui se situe dans la plage allant de 170 °C à 250 °C et à une pression qui se situe dans la plage allant de 0,1 MPa à 1,0 kPa.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03029339 A
- WO 2003029180 A1
- US 5286898 A
- US 5319129 A
- DE 2823165
- US 3027398 A
- WO 0078704 A
- DE 10161010
- US 20030069135 A1
- WO 2004009526 A1
- EP 1042273 A
- DE 10116812
- EP 1314714 A
- EP 0814098 A
- DE 10225565
- DE 10232868
- US 6284938 B
- US 6080903 A
- US 6072093 A
- US 6025533 A
- US 5990367 A
- US 5895830 A
- US 5856604 A
- US 5847252 A
- US 5081086 A
- EP 0395857 A
- EP 1171413 A
- EP 0850905 A
- EP 1172349 A
- WO 2004020380 A
- DE 10327435
- EP 1186593 A
- EP 1300388 A
- DE 102005021075
- WO 2004046078 A

**In der Beschreibung aufgeführte Nicht-Patentliteratur**

- Ullmann's Enzyklopädie der Technischen Chemie. 1986, vol. 5, 254-260